# EUROPEAN PATENT APPLICATION

(11) **EP 3 839 961 A1**
(43) Date of publication of application: **23.06.2021**
(21) Application number: 19850094.4
(22) Date of filing: 13.08.2019
(51) Int. Cl.: G16H 10/65, G16H 40/20, H04W 4/80

(54) **SYSTEM AND METHOD FOR MONITORING AND MANAGING INTERACTIONS BEING HUMAN BEINGS AND/OR INANIMATE BEINGS**

(30) Priority: 13.08.2018 BR 102018016532
(71) Applicant: Tozatto, Marcelo Goulart, 20010-121 Rio de Janeiro (RJ) (BR); Tozatto, Arthur Henrique Goulart, CEP: 04512-001 São Paulo (SP) (BR)
(72) Inventor: Tozatto, Marcelo Goulart, 20010-121 Rio de Janeiro (RJ) (BR); Tozatto, Arthur Henrique Goulart, CEP: 04512-001 São Paulo (SP) (BR)
(74) Representative: Hoefer & Partner Patentanwälte mbB
(86) International application number: PCT/BR2019/000025
(87) International publication number: WO 2020/034014

(57) **Abstract**

A system and method of monitoring and managing interactions between living things and / or lifeless things is described, comprising two or more electronic devices attached to things (DIU and/or DIE) that interact with each other through communication protocols, or just they read emitted signals, without necessarily establishing communication, upon of physical proximity that establishes the reception range of the signal, and sending these interactions, through communication protocols, to a central processing unit (CPU) where the data are analyzed and processed by a Event Generating Process (EGP) which compares data previously registered in databases and forwards it to a Server Process (SP) equipped with artificial intelligence for decision making based on the analysis of data and events collected and stored in database of central processing unit (CPU), called Server Process (SP) that communicates with electronic devices (DIU) and (DIE) by communication protocols.

## Description

### THE FIELD OF INVENTION

The present invention patent describes a system and method of monitoring and managing interactions between living things and / or lifeless things that has an electronic device attached, which determine events of interactions between things (living things and lifeless things), allowing to monitor, control and record the interactions in a central processing unit which selects, processes, classifies, analyzes and records the events, returning with necessary commands or information that support decision-making processes related to living things and / or lifeless things being monitored.

### BACKGROUND OF THE INVENTION

Systems and methods for monitoring living things and / or lifeless things generally make use of geographical position references by trilateration, through fixed routers of known position, making the solution complex, very costly and inefficient, with no condition to identify interactions between living and / or lifeless things.

Alternatively, antennas are used that induce a magnetic field in a coil that then returns a signal to them for identification, resulting in a high-cost solution, complexity in implantation, with a field limited to the surroundings of the antenna (which would imply the implementation large number of antennas for a minimum effective coverage), or the identification via NFC technology (Near Field Communication), which requires a few centimeters proximity to establish any communication between two devices, which, in the same way, would imply in high cost with low efficiency and flexibility.

In the case of monitoring systems in healthcare, today are made from a very manual way, with minimal technological controls and a great human intervention, which takes time to the proper records as well as expands the margin of error due to human errors inherent in the process.

It is estimated that 70% of professionals working time at the hospital bedside is spent on processes, validations and control notes. In many health institutions, the administration of medications is still analogical, where after prescription, each patient starts to be identified through a spreadsheet where the medications, dosage and other complementary guidelines are noted.

According to data published by the Institute for Supplementary Healthcare Studies at the end of 2017, about 829 Brazilians die daily due to failures in healthcare routines, both in public and private hospitals, which is equivalent to 3 deaths every 5 minutes.

Therefore, as in other areas of knowledge, the establishment of routines and precision in their execution is a required condition, in order to avoid any human error that causes damage to the health of the user. The state of the technique describes several systems and methods that monitor and manage medical routines.

The document US6571193 describes a method, apparatus and system for automatically recognizing movements and actions of moving objects, such as humans, animals and machines. Measuring instruments are attached to an object under observation to measure a change in status that implies the movement or action of the object and to emit a signal indicating the measurements. A characteristic quantity extraction unit extracts a characteristic quantity from the received measurement signal that represents the movement or action currently performed by the object under observation. A signal processing unit for motion / action recognition correlates the extracted characteristic quantity with reference data in a database containing previously acquired characteristic quantities of movements and actions. The movement or action represented by the characteristic quantity with the highest degree of correlation is recognized and produced.

The document US20170173261 describes a system and method for automatically delivering a particular medication to a user. A sensor attached to a user can collect information about the user. A controller can use the information collected to determine an amount of medication to deliver to the user. The controller can instruct a medication delivery device to dispense medication to the user. The controller can be part of or implemented in a cell phone.

The document CN204181603 describes a nursing monitoring device that can be tracked in real time, based on the hospital environment, which comprises a signal selector used to capture vital sign information of body temperature, respiration, blood pressure and blood oxygen, a code recorder two-dimensional bar code to record patient information, an information analysis device used to process and monitor information, a wireless receiver transmitter used to transmit information from the signal picker and two-dimensional bar code recorder to the data analysis device information. The two-dimensional picker and barcode recorder are connected to the input port of the wireless receiver transmitter, and the information analysis device is connected to the output port of the wireless receiver transmitter. The signal picker captures the signals from a patient's vital parameters, the information analyzer device processes and monitors the signals from the patient's vital parameters, the signals from the patient's vital parameters no longer need to be captured manually.

The document US9215577 describes a wearable healthcare device that includes a support for a care receiver. The support is provided with means of operation, means of detection, means of positioning, means of Bluetooth communication, means of help call, means of wireless communication, means of wireless transmission and means of information processing. The information processing means generates health information for the doctor using the remote server according to the information in the above means. The information processing medium receives the health information, which is from the remote server, which analyzes the information previously sent, to remind the care receiver appropriately.

The document US20150324528 describes a system and method for tracking patient service provider interactions. In some implementations, a method includes collecting information almost in real time about the patient's interactions with staff in health facilities, such as hospitals, nursing care facilities, retirement communities, home care environments, assisted living facilities and facilities for the elderly. Patient interaction information can be used to help ensure that accurate healthcare information is collected from interactions between patients and healthcare professionals using a wearable identification transceiver with a built-in microprocessor system, as well as an integrated RFID reader and an RFID tag.

The document WO2010108287 describes a system and method to monitor a user's health and provide feedback, comprising physiological sensors, activity sensors, a processor, a real-time detection and analysis module for continuous monitoring of activity and health, adjusting user mode with adaptive optimization, capacity data collection to record important health information, audio outputs for the user via audio path and audio interface, alarm conditions predefined and confirmed by the user via wireless communication network for the appropriate individual for immediate assistance and needed. The system uses non-invasive monitoring technology for continuous, painless and healthless monitoring.

WO2008098346 describes a system and method comprising one or more vital signal sensors, activity sensors, a real-time detection and analysis module for continuous health monitoring, intelligent audio outputs and speech advice for the user via audio paths and audio interface.

Despite the accuracy in drug administration, the state of the art monitoring systems are strongly focused on a specific relationship of access to databases and verification of information, without being able to monitor, track and establish routines and processes based on interactions both of users with other users and or with lifeless things, in order to allow verifying not only if a certain item was made available to the user, but also to allow monitoring the conditions of this user at the moment of a certain action (such as at the moment of administering a drug), among other aspects that are essential for security as well as in a process auditing system.

Thus, the object of the present invention patent is a system and method of monitoring and managing interactions between living things and / or lifeless things with electronic devices that establish interactions based on the recognition of an active identification code, allowing to track, monitor, alert, certify and record activities among the various users (living things and / or lifeless things), providing information and online instructions for carrying out the activities and, additionally, allowing a third party to follow in real time the actions and interactions between living things and / or lifeless things, allowing to generate alerts about actions not related to the standard of protocols.

### SUMMARY

It describes a system and method of monitoring and managing interactions between living things and / or lifeless things that identifies and stores all interactions that occur between electronic devices attached to the various living things and lifeless things that integrate the system, by proximity.

It describes a system and method of monitoring and managing interactions between living things and / or lifeless things that identifies and records the distance between electronic devices attached to the various living things and lifeless things that integrate the system.

It describes system and method of monitoring and managing interactions between a living things and / or a lifeless things potentially useful for the care of patients in a hospital environment, by the introduction of artificial intelligence in the collection, certification, registration and analysis of information, as well as such as sending and directing guidance for procedures and processes in an automatic, conditional and individual manner to the employees involved in the service, providing greater patient safety by reducing the risk of error, reducing the operational cost and optimizing the time of professionals dedicated to the processes of attendance.

It describes system and method of monitoring and managing interactions between a living things and / or a lifeless things, which identifies all interactions between electronic devices attached to living things and lifeless things in the system, allowing to identify and monitor ongoing care, the professional who is providing the service, the patient being served, and the length of service, among others, generating data for the optimization and improvement of the bedside care teams, for example.

It describes a system and method of monitoring and managing interactions between living things and/or liveless things that allows sending information online about patient care to caregivers and family members visiting the hospital.

It describes a system and method of monitoring and managing interactions between living things and / or lifeless things that identifies all interactions between living things and / or lifeless things with attached electronic devices, making it possible to track and identify everyone who have had contact, and for how long, with something alive and / or lifeless with attached electronic devices and suspected of contagious contamination, and may generate some type of safety instruction to be followed immediately.

It describes a system and method of monitoring and managing interactions between living things and / or lifeless things , whose platform allows the reading of barcodes or QR codes, widely used in the detection of objects or identification of persons, reducing some impact for the implementation of the new technology described here.

It describes a system and method of monitoring and managing interactions between living things and / or lifeless things that allows certifying the user's identity, in order to authorize or restrict their access, whether in physical restraints (such as turnstiles, gates, doors), among others) or in systems, being able to log in by the simple proximity between an electronic device attached to a living thing (DIU) and an electronic device attached to a lifeless thing (DIE), as well as logging out by simple distancing.

It describes a system and method of monitoring and managing interactions between living things and / or lifeless things that allows the living thing with an attached electronic device to trigger an emergency using the electronic device itself.

It describes a system and method of monitoring and managing interactions between living things and / or lifeless things , which guarantees the dynamic updating of control records and processes, without the need for human intervention.

It describes a system and method of monitoring and managing interactions between living things and / or lifeless things with potential application in the constant monitoring of physical and biological parameters of greater risk of accident, such as falls and sudden variations in body temperature, sending alerts to electronic devices (DIU) attached to previously listed users, providing the ability to promptly respond in case of falls or adverse reaction to some medication, for example.

It describes a system and method of monitoring and managing interactions between living things and / or lifeless things that identifies all interactions between electronic devices attached to living things and / or lifeless things, even recognizing the distance between them, which allows you to monitor lifeless things , by registering the configuration and / or setup performed, managing the information in the Monitoring Unit database, allowing the supplier and / or manufacturer to remotely monitor and analyze the performance, assertiveness and quality of lifeless things and its correct use in operation, identifying deviations, failures or potential defects. This increases patient safety as the equipment manufacturer itself identifies a possible malfunction, often not noticed by the user, being able to anticipate preventive maintenance or remove equipment from operation that has recurring problems, as well as check the productivity of the equipment in operation.

It describes a system and method of monitoring and managing interactions between living things and / or lifeless things that identifies all interactions between things (DIUs and or DIEs) with attached electronic devices, allowing control of medication administered to a patient.

It describes a system and method of monitoring and managing interactions between living things and / or lifeless things that allows monitoring patients under the attention of a residential health care company (Home Care), through an artificial intelligence system capable of monitoring biological parameters, physiological and interactions, sending all the collected data to a monitoring unit that analyzes the data, records the events and allows the emergency response in case of accidents (falls, cardiac arrest, arrhythmias, high fever, routine deviation, among others), treatment monitoring and medication taking, certification, validation and monitoring of care provided by caregivers and health professionals.

It describes a system and method of monitoring and managing interactions between living things and / or lifeless things with potential application in monitoring the elderly, maintaining constant care and continuously monitoring biological, physiological parameters and interactions, sending the obtained data to pre-registered recipients, providing the emergency response capacity in case of accidents, monitoring of treatments and taking medication, certification, validation and monitoring of care provided by caregivers, doctors, physiotherapists, nurses, etc.

It describes a system and method of monitoring and managing interactions between living things and / or lifeless things that records interactions at major events between visitors and exhibitors, allowing the identification of the interaction time and the appropriate records of the profiles, allowing the exchange of data, such as exchanging virtual business cards at events.

It describes a system and method of monitoring and managing interactions between living things and / or lifeless things that allows implementing the emergency response capacity to an adverse user safety event, without the need for user intervention, identifying physiological changes that may cause some type of risk to the health of the user, allowing the system to identify the risk situation and generate an alert to the central processing unit that can trigger third parties qualified in the system and / or third parties close to the user at risk, from interaction of electronic devices attached to users.

It describes a system and method of monitoring and managing interactions between living things and / or lifeless things that allows the user to be alerted with an attached electronic device about the medication schedule, according to the medical prescription, exams and procedures, as well as to the health professional who also has an electronic device attached.

It describes a system and method of monitoring and managing interactions between living things and / or lifeless things that allows you to attach an electronic device to a Supply Distribution Compartment (CDI), which can generate alerts about the expiration date or any other relevant information to any electronic device attached to a user who approaches this electronic device attached to the CDI, such as suspended medication alert, medication scheduling and rescheduling automation, medication temperature curve control, formulated drug stability control, among others .

It describes a system and method of monitoring and managing interactions between living things and / or lifeless things that allows you to record an interaction between an electronic device attached to a Supply Distribution Compartment (CDI) with drugs and an electronic device attached to a user, automatically recognizing whether a drug foreseen for a patient should not be administered because the patient is allergic to that medication, even if it is a non-prescription drug, sending the alert to the professional.

It describes a system and method of monitoring and managing interactions between living things and / or lifeless things that allows the control of drug dispensing, allowing to track the Supply Distribution Compartment (CDI), its contents, the professionals who handled it and the patient who was treated with the drug.

It describes a system and method of monitoring and managing interactions between living things and / or lifeless things that allows you to track critical drugs, such as psychotropics, and other controlled drugs, tracking all living things and / or lifeless things that came close to them, and if the storage procedures were followed, as well as the user responsible for opening the Supply Distribution Compartment (CDI), it was authorized, generating alerts when the opening was identified by an unauthorized user.

It describes a system and method of monitoring and managing interactions between living things and / or lifeless things that allows the schedule of the doses of each drug to be automatically scheduled by identifying the time of the first dose, from which the system schedules the next doses and defines , including the script and sequence that must be followed by the person responsible for administering the drugs in order to maintain the correct time interval between doses for each patient. In the same way, the system dynamically adjusts this script to be followed by employees according to the information that is collected with the times of this operation at the bedside, optimizing the allocation of teams of employees and always ensuring the correct scheduling of drugs.

It describes a system and method of monitoring and managing interactions between living things and / or lifeless things, that is able to take decisions in an automatic and dynamic way in relation to rescheduling, missed dose and time tolerance, according to the respective protocols. These same protocols can be fed back and improved by the system, since it will be monitoring and controlling all the movement of the bedside, identifying and recording all the execution and any deviation. A system here that the more it is used the more it learns and applies this learning in benefit of the constant improvement of the protocols.

It describes a system and method of monitoring and managing interactions between living things and / or lifeless things that allows you to keep track and track drugs even after you leave and until you return to the pharmacy. In this way, it expands drug storage locations beyond the limits of the pharmacy, creating the possibility of decentralized dynamic stock through constant control and monitoring of Supply Distribution Compartment (CDI) attached to an electronic device (DIE) throughout the process until the returns to pharmacy.

It describes a system and method of monitoring and managing interactions between living things and / or lifeless things that, through an electronic device attached to a Supply Distribution Compartment (CDI), indicates and monitors the return of unused drugs returned to said Compartment (CDI), the return to the pharmacy, controlling and avoiding the intentional or unintentional diversion of any item. Each drug returned to CDI, records the reason for its return in the system and this information is used in the pharmacy for the destination of these returned drugs.

It describes a system and method of monitoring and managing interactions between living things and / or lifeless things , which through an electronic device attached to the Supply Distribution Compartment (CDI), is able to identify each user with an attached electronic device that at some point conducted this compartment, assigning it the information and the responsibility of delivering it to its destination, both when going to the patient and when returning to the pharmacy.

It describes a system and method of monitoring and managing interactions between living things and / or lifeless things , which alerts even before a supply is incorrectly administered (such as a drug) through the interaction of a supply with an attached Electronic Device and a patient with an Electronic Device attached, increasing patient safety.

It describes a system and method of monitoring and managing interactions between living things and / or lifeless things , potentially useful for drug administration in hospitals, for example, where an electronic device is attached to a Supply Distribution Compartment (CDI) or to a specific Supply (like a drug) and is linked to a specific patient, who also has an electronic device attached, signaling the 'right patient' and the 'correct time' for opening, linked to the proximity of the right patient at the right time where drugs should be administered.

It describes a system and method of monitoring and managing interactions between living things and/or lifeless things , which, through an electronic device attached to a Supply Distribution Compartment (CDI) is able to indicate a wrong patient, blocking the opening of the Compartment (CDI) and / or generating an alert.

It describes a system and method of monitoring and managing interactions between living things and / or lifeless things, which generates a database that allows the comparison of events and the analysis of their efficiency and effectiveness. For example, the different protocols defined for each drug in different health units can be analyzed, based on the results of the patient's evolution in relation to each protocol, by comparing the data collected automatically and related to the events of each patient and their reactions.

### BRIEF DESCRIPTION OF THE FIGURES

Figure 1 shows the schematic representation of the system for monitoring and managing interactions between living things and / or lifeless things.

Figure 2 shows the interaction flowchart between electronic devices attached to users (DIUs) and / or electronic devices attached to lifeless things (DIEs).

Figure 3 shows the flowchart of authentication and access of electronic devices attached to users (DIUs) and / or electronic devices attached to lifeless things (DIEs).

Figure 4 shows an example of interaction between different things attached with DIUs and DIEs, with steps to verify interaction, monitor interaction and the opening authorization of an electronic seal for a supply (or drug) distribution compartment (CDI).

Figure 5 shows the flowchart of the medication dispensing steps, evidencing the interaction of a DIE attached to a Supply Distribution Compartment (CDI) with a DIU attached to a patient and with an DIU attached to the user who administers the medication.

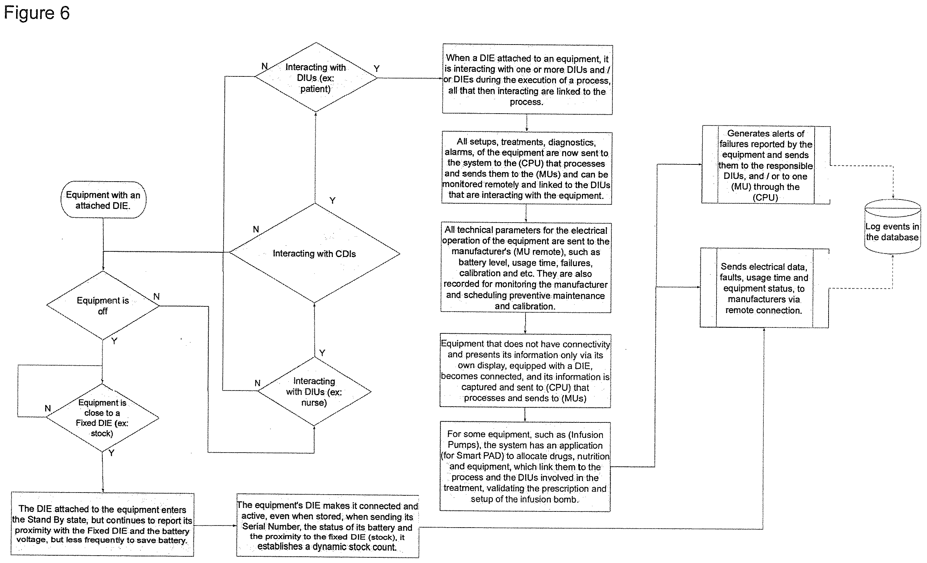

Figure 6 shows the flowchart of a DIE attached to a lifeless thing to obtain data to be sent to the central processing unit (CPU).

Figure 7 presents the example flowchart of the monitoring operations of a DIE attached to an infusion pump.

Figure 8 shows the flowchart of operations involving the monitoring of bodily and extracorporeal aspects of the user with a DIU.

Figure 9 shows the flowchart of the interactions between DIEs and / or DIUs and the physiological factors of the users or group of users, the extracorporeal factors of an environment and the relationships with adverse safety events.

Figure 10 shows the flowchart of interactions between DIUs and / or DIEs applied in an event environment (such as fair and exhibition), showing the capacity for statistical survey and relationships established between visitors, exhibitors, exhibited equipment and stands.

### DETAILED DESCRIPTION OF THE INVENTION

For the purposes of the present invention patent, the following terms are conceptualized:

"Thing" is used in a generic way to designate any living thing (humans, animals, or any other living organism) or lifeless thing (objects, equipment, structures, places, ...) with an attached electronic device, which establishes the interaction with other electronic devices attached with other living things or lifeless things;

"lifeless thing" is used to designate an inorganic material element, like: objects, equipment, structures, places, package, instruments, etc.

"electronic device" is used to designate a physical structure to be attached to a living things or lifeless things that features an electronic circuit board equipped with a wireless communication module that sends and receives signals, through communication protocols, with other electronic devices and/or with a central processing unit, said electronic device with a power source, a processor, memory, and sensors configurable according to the application, which may present a visual interface type display.

the acronym "DIU" and its plural "DIUs" will be used in the scope of the present invention patent to reference an electronic device attached to a user (living thing);

the acronym "DIE" and its plural "DIEs" will be used in the scope of the present invention patent to reference an electronic device attached to a lifeless thing;

the acronym "fixed DIE" means an electronic device attached to a static lifeless things ;

"central processing unit (CPU)" comprises at least one computer, with at least one processor, one storage environment to the registration of things, the registration of associations between things, the registration of associations between things and DIUs and or DIEs, the recording of interactions between DIUs and or DIEs, the recording of sensor information from DIUs and or DIEs, an Event Generating Process (EGP), a Server Process (SP) and a set of instructions that perform the decision-making processes according to the protocols deployed and synchronize the databases to make data access available locally or remotely.

"Monitoring Unit (MU)" comprises a computer that receives data from the central processing unit (CPU) and / or from a Server Process (SP) to allow the monitoring of interactions and other alerts generated in the system.

"Supply Distribution Compartment" (CDI) comprises a physical structure necessarily with an attached DIE used to package one or more supplies, and features an electronic seal for opening and closing control.

"Application" comprises all types of software used for system operation, which are optionally used in tablets, personal computers, smartphones, smart pads, among others, since they have a DIE attached or be able to use their own operating system, through API, to communicate with electronic devices (DIEs) and (DIUs) and with the Central Processing Unit (CPU) and have the ability to achieve all the features described in the present invention. They are used for monitoring, certification, data entry and collection, management, control and monitoring of the processes described in each of the functionalities.

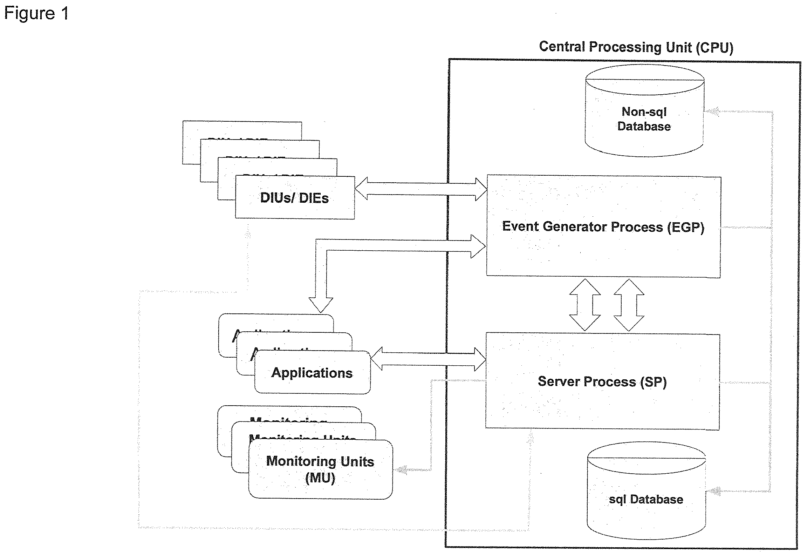

As shown in figure 1, the system for monitoring and managing interactions between living things and / or lifeless things comprises two or more electronic devices attached to things that interact with each other through communication protocols, or only read emitted signals, without necessarily establish communication, through physical proximity that establishes the reception range of the signal, which can be, at least, two electronic devices attached to users (DIUs), two electronic devices attached to lifeless things (DIEs) or one electronic device attached to user (DIU) and an electronic device attached to an lifeless thing (DIE), this messages with information of the read signals that are sent, through communication protocols, to a central processing unit (CPU) where the data are analyzed and processed in a Generating Process of Events (GPE) that compares and submits the registered logic and defines this interactions between the things and records in the databases, which is accessed by the Server Process (SP) equipped with artificial intelligence for decision making based on the analysis of previously registered data and events collected and stored in the central processing unit (CPU), said Server Process (SP) that communicates with electronic devices (DIU) and (DIE) through communication protocols.

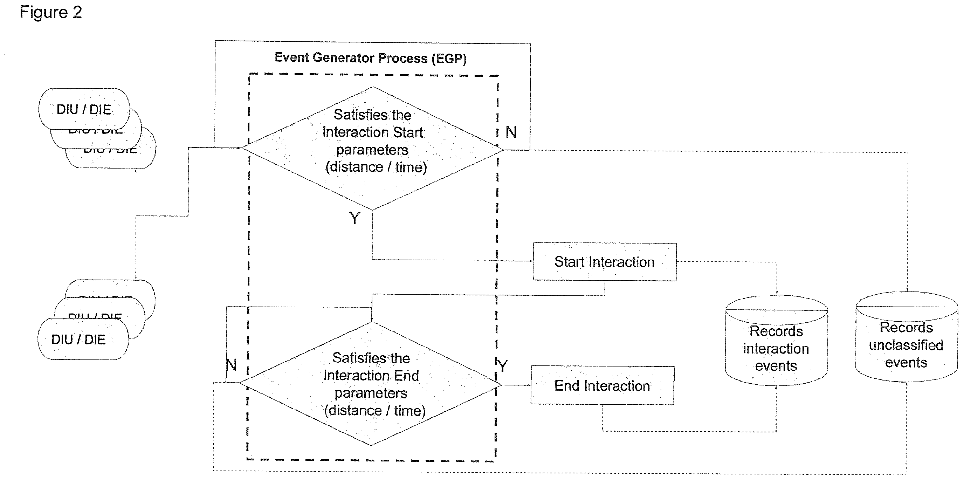

As shown in Figure 2, the interactions established between the electronic devices attached to a thing (DIU) and (DIE), defined by physical proximity of the devices (DIU) and/or (DIE), allows establishing the interaction time, and the distance between them, said records of interactions, stored in the central processing unit (CPU). Records stored in the Central Processing Unit (CPU) can be associated with previously stored data in order to promote links (relational database), or to generate a non-relational database where unclassified events are recorded.

Optionally, a DIE is attached to a conventional electronic device, such as tablets, personal computers, smartphones, smart pads, among others.

Optionally, these conventional electronic devices communicate with electronic devices (DIEs) and (DIUs), or at least can read their signals, without need to establish communication, and communicate with the Central Processing Unit (CPU) through their own operating system or through API "Application Programming Interface", allowing to follow, certify, collect data, manage, control and monitor the processes described in each of the functionalities, through automatic login through the interaction of the electronic device attached to the user (DIU) with the conventional electronic device or with the electronic device (DIE) attached to said conventional electronic device, as described in the previous paragraph.

In the context of the present invention, the term interaction comprises the physical proximity between electronic devices (DIUs) and / or (DIEs) for a specific minimum time. Electronic devices (DIUs) and (DIEs) have the ability to read the intensity of a signal emitted by another electronic device (DIU) and / or (DIE) that are within reach. Each electronic device (DIU) and / or (DIE) captures this information from all other electronic devices (DIUs) and / or (DIEs) that are within reach, format and send them to the Event Generator Process (EGP) of Center Processing Unit (CPU), where the information received is submitted to a set of instructions that defines between which electronic devices (DIUs) and / or (DIEs) should be considered that there is a beginning of interaction, and later, in the same way, submitting to another set of instructions that checks and defines whether there is an end of interaction and how much time has elapsed between the beginning of an interaction and the end of this interaction. All interactions are formatted and forwarded to a relational database, and those that were not defined as interaction are formatted and forwarded to a non-relational database. The Server Process (SP) accesses the relational database, processes and analyzes the interaction information and acts according to the system directives, returning information, commands, actions and instructions to the electronic devices (DIUs) and / or (DIEs)), applications and Monitoring Unit (MU).

Electronic devices (DIUs) and (DIEs) are a physical structure consisting of: identification code (ID) with an electronic circuit board, a processor, a wireless communicator, sensors, firmware and a power source. The electronic device (DIU) and (DIE) have physical means that allow it to be attached to a user (living thing) or to a lifeless thing, such as handles, straps, clips, among others.

The identification code (ID) of each electronic device (DIU) and (DIE) is associated, respectively, with the profile of the user who will has said electronic device (DIU) attached or with the characterization of the lifeless thing to said electronic device (DIE) will be attached, these associations being registered in the databases at the Central Processing Unit (CPU) through an application.

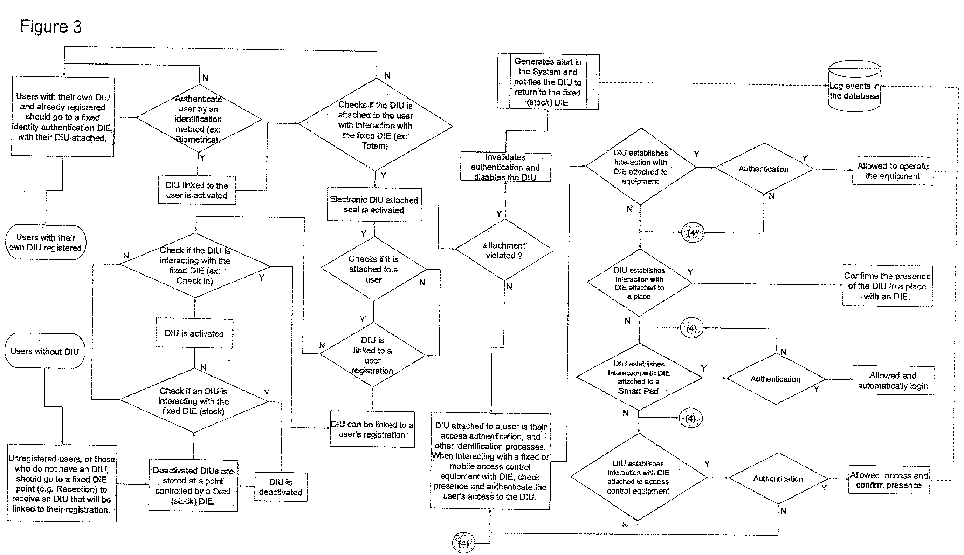

As shown in figure 3, the linking of a thing to an electronic device (DIU) and / or (DIE) is done through an application, which contains the thing's registration and the identification code (ID) of the electronic device (DIU) and / or (DIE), this link held in a specific location that contains a fixed electronic device (Fixed DIE), registered in the system as a registration and device link point, and performed by a professional responsible for the registration, which has a DIU with registration authority. The electronic device (DIU) or (DIE) already linked is activated when said device (DIU) or (DIE) is attached to the thing and establishes an interaction with the registration and device link point.

Optionally linking a living thing or lifeless things to an electronic device (DIU) or (DIE) is done through a self attendance totem equipped with a Fixed DIE, registered in the system as an automatic device registration and linking point. In this self attendance totem, a living thing already registered in the system and with the DIU attached, but said DIU without authenticity proved, can done the association through a method of personal recognition such as, but not limited to, biometrics, maintaining the integrity of the link proven by personal identification, as shown in figure 3.

Electronic devices (DIU) and (DIE) are equipped with decoupling sensors, such as optical, capacitive, luminance, infrared or mechanical sensors, whose function is to identify the removal or uncoupling of an electronic device (DIU) or (DIE) of your thing. The decoupling sensor has the function of making the binding of an electronic device (DIU) or (DIE) non-transferable, invalidating, when decoupled, the authentication capacity, and generating alerts to devices registered in the system to receive decoupling and invalidation alerts.

Optionally, the electronic devices (DIU) and (DIE) have an emergency triggering means, such as a physical button, by sensor or made available on the interface of the electronic device (DIU) or (DIE), said emergency triggering means that sends a request for assistance to other electronic devices (DIUs) and / or (DIEs) and / or to a Monitoring Unit (MU), according to the routine established in the Central Processing Unit (CPU).

Optionally, electronic devices (DIUs) are equipped with sensors of physiological parameters, such as: heart rate, body temperature, blood pressure, glycemic index, which are linked to the user with the electronic device (DIU) and monitored by the Central Process Unit (CPU), which generates alerts to other devices (DIUs) and (DIEs) when identifying changes in the parameters, according to a pre-established standard. These physiological parameters sensors allow to associate the monitored parameters of the patient with an electronic device (DIU) to bedside care, alerting about possible changes after a procedure, such as taking drugs.

Optionally, electronic devices (DIUs) are equipped with accelerometer, like motion sensors, that send movement and free fall data to the Central Processing Unit (CPU) that processes and analyzes the data received, comparing it with other user parameters and with the standard behavior routine, in order to verify potential normality and potential abnormality.

Optionally, the electronic devices (DIU) and (DIE) have an audio-visual interface, leds, microphone, speaker, which serve as a means of interacting with the user of an electronic device (DIU) or (DIE) to send and receive actuation, information, instructions, audible and / or visual alerts. As an example, the user can indicate on the interface, on a predefined scale, the level of pain perception after the administration of a medication, or report his perception of improvement during a treatment, said data that are received and processed by the CPU that generates a database capable of automatically defining which of the treatment protocols is most effective and suggesting changes based on the collection of data imputed by the user and collected by the DIUs and DIEs. DIUs can receive messages from scheduling procedures and exams.

The electronic devices (DIUs) and (DIEs) have the ability to define the relative distance between them and the time spent in each distance, this data is stored in the CPU and thus allowing to track and identify all users with DIUs that eventually they have been at a certain distance, for a certain time, from an DIU attached to a potentially contagious user, allowing to select and identify all users with a potential risk of contamination.

Electronic devices (DIUs) and (DIEs) that use batteries as a source of energy, have implemented an automatic energy saving system, activated in specific situations, such as when maintaining interaction with fixed DIE defined in the system as a storage point. In this situation, the electronic devices (DIU) and (DIE) go into reduced operation mode, disabling most of the functions to save battery, maintaining only the interaction and communication recognition functions, but with longer capture and transmission intervals.

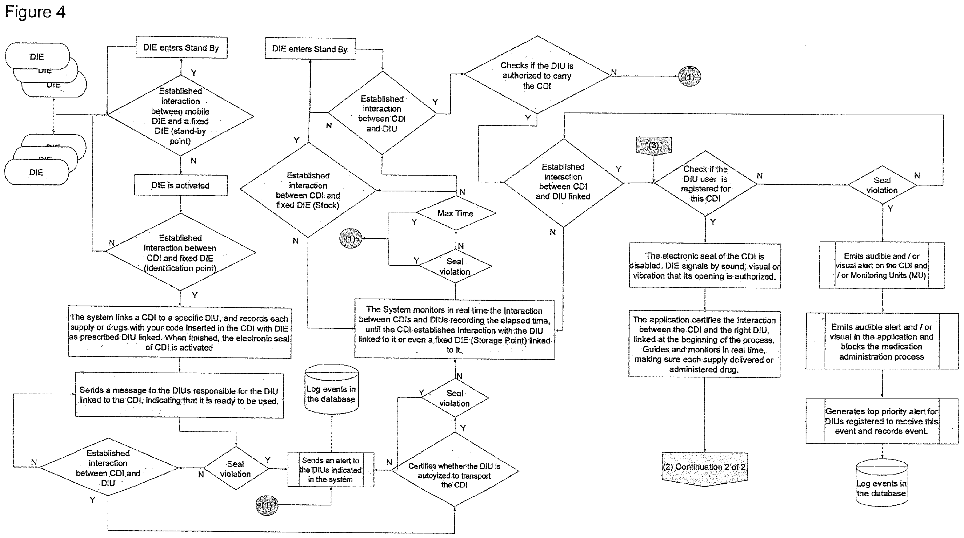

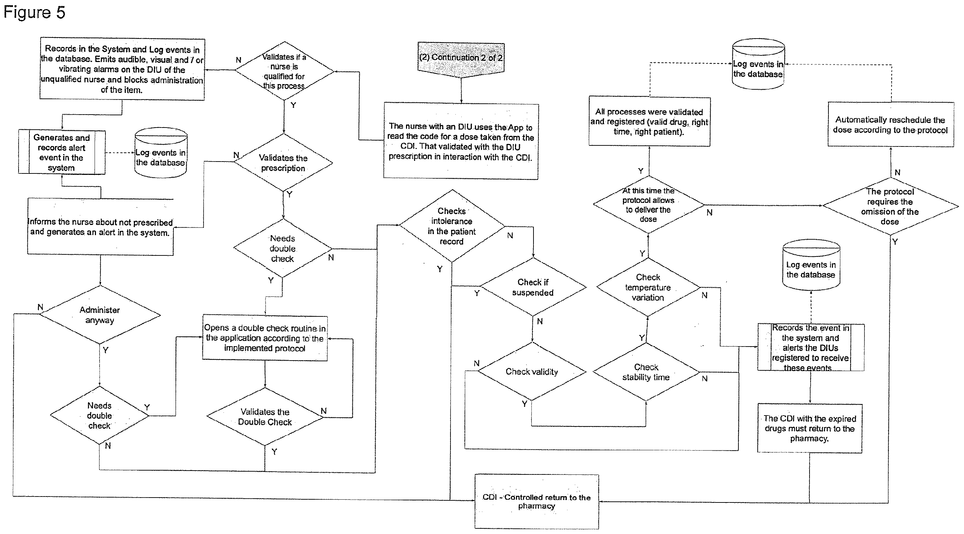

As shown in figures 4 and 5, supplies to be administered to users can be stored in a Supply Distribution Compartment (CDI) or can have an attached DIE. In both situations, the supplies have their properties monitored, as well as the storage conditions, whose events are recorded in the database of the Central Processing Unit (CPU) and, in case of non-conformity, being able to generate alerts to one or more electronic devices (DIUs) defined in the CPU.

In the first situation, that is, if the supplies are stored in a Supply Distribution Compartment (CDI), the supply or a group of supplies is identified by a readable identification code through a reader, which contains information about the expiration date, temperature, among others. The link of the supply to the CDI and the link of the supply to the user to whom the supply is intended (with a DIU attached) is registered in the Central Processing Unit (CPU) by the sector responsible for the distribution of supplies, for example, through access to the system and registration of this association, as well as specifics of the supply, such as prescribed dose, administration time, said sector of distribution sector being identified by means of a fixed electronic device (Fixed DIE).

Thus, the interactions between the electronic device (DIE) attached to the supply, the electronic device (Fixed DIE) installed in the physical space of the supplies distribution area, the electronic device (DIU) of the user who performed the prescription registration at the Central Processing Unit (CPU) and the electronic device attached to the user (DIU) linked to the supply are registered at the Central Processing Unit (CPU), allowing the tracking of information.

Established the end of interaction between the electronic device (DIE) attached in the Supply Distribution Compartment (CDI) and the Fixed DIE installed in the supply distribution sector and registered as such in the Central Processing Unit (CPU), and being certified that the state of the CDI electronic seal is closed, the Central Processing Unit (CPU) generates an alert to electronic devices (DIUs) linked to the user receiving the supply (with an DIU attached) in order to indicate the availability of the supply. The CDI with the attached electronic device (DIE) is transported by a user with an electronic device (DIU) attached and enabled for this transport, this interaction being registered at the Central Processing Unit (CPU), which can even monitor the movement through the locations that have electronic devices (fixed DIEs) installed at certain points on the route. If the Supply Distribution Compartment (CDI) is moved by a user with an attached electronic device (DIU) not enabled for this event, the Central Processing Unit (CPU) generates an alert. Likewise, the CPU generates an alert if there is a physical distance between the electronic device (DIE) attached to the CDI and the electronic device (DIU) authorized for the transport of that CDI, unless said DIE has already established interaction with one or more (Fixed DIE, or patient with DIU) specified as the destination of this DIE.

The approach of the CDI containing the electronic device (DIE) with the electronic device (DIU) attached to the patient linked to that CDI generates a correct patient certification (that is, the DIU of the patient associated with the CDI's DIE) or an alert in case of a wrong patient (that is, the patient's DIU not associated with the CDI's DIE).

The Event Generator Process (EGP) checks the interaction between the patient's electronic device (DIU) and the CDI's electronic device (DIE) and records it in a database. The Server Process (SP) checks whether said electronic devices (DIU) and (DIE) are associated and whether the time of the interaction is in accordance with that registered in the specific database. If the data are related, the Server Process (SP) authorizes the opening of the electronic seal of the Supply Distribution Compartment (CDI).

The central processing unit (CPU) records the interaction between the electronic device (DIU) attached to a user who conducts the CDI, the electronic device (DIU) attached to the patient and the electronic device (DIE) attached to the CDI. When the opening of the electronic seal of the CDI is authorized, the electronic device (DIU) attached to the user authorized to administer the supply to that patient (with a DIU attached) can receive guidance on the administration of the supply, through the interface provided on the electronic device (DIU) attached. When administering the supply (in this case, a drug) to a patient with an DIU attached, the readable code presented with the supply is read, which can generate alerts for expired expiration dates, for example, said code read by through the application installed on a mobile device that may have an electronic device (DIE) attached if it is unable to interact with electronic devices (DIUs) natively.

In the situation when the supply has an electronic device (DIE) attached, the same necessary steps of binding and tracking are required, as already described in the situation of the supply being deposited in a CDI.

In the event that the supply requires a supply routine to a specific user, this routine is registered at the CPU. For example, when the supply is a medication, the system registers the routine of distributing the first dose of a group of prescriptions to their respective patients, processes the information collected by the established interactions, times and deadlines and automatically calculates the next time according to the interval specified for each drug in each prescription, as well as calculating how long it will take to perform the same routine. In addition, the system generates and sends medication schedule alerts to all electronic devices (DIUs) and (DIEs) involved in linking, to ensure that the distribution is made at the times specified in the prescription.

The rescheduling of the administration of a supply is necessary whenever it is not delivered according to the schedule of the prescription (including the grace period) to the recipient of the supply. For example, a drug that will be administered by a professional (with an electronic DIU device attached) to a patient (with an electronic DIU device attached) with 4 hours delay, when the interaction between those involved in the event is established, the CPU alerts that the drug is being administered more than 4 hours late and, according to the protocol references registered for this drug at the CPU, the recommendation is the administration of the dose, however the subsequent doses must respect the interval between doses initially prescribed. Thus, the system automatically defines the new scheduling of the next doses considering the time of administration of the dose administered with delay.

In the case of an attempt to administer a non-prescribed supply to a specific patient, the Central Processing Unit (CPU) identifies the interaction between the DIE attached to the CDI or to the supply, the electronic device (DIU) of the user who dispenses the supply and the electronic device (DIU) of the patient who will receive the supply and generates a non-prescription alert, providing a pending prescription option. In this situation, and with the interaction of the electronic devices (DIUs and DIEs) involved is maintained, the Central Processing Unit (CPU) generates an alert and presents the need for confirmation of supply administration in the application or in the interface of the electronic device (DIU) of the responsible user for administering the supply and triggers a protocol defined in the system, which may, for example, require authorization from the doctor responsible for the patient or even prevent the administration of non-prescriptions.

The system can alert if a drug (with a DIE attached or contained in an CDI) is on the verge of being administered to an intolerant user, sending an alert through the interface of the electronic devices (DIU) and / or (DIE) and / or application.

Supplies that require specific routines for storage, transportation and / or administration can have their specific routines registered in the system. This category of supplies, previously identified in the CPU database, may for example require the need for Double Check, sending an alert through the interface of the electronic device (DIU) and (DIE) and / or application, as well as presenting in this interface the routine to be followed and that must be confirmed by the user receiving the alert. For example, a drug that can cause serious consequences if administered in the wrong way, can be categorized as a supply that requires a specific routine, as shown in figure 5.

The system allows to control, monitor and record the return to the stock of any supply that has not been used for any reason, registering the return at the CPU and being able to generate an alert for information of the reason.

One or more DIEs can be coupled to equipment such as infusion pumps, allowing to identify interactions, identify who prepared an infusion, which patient is receiving the infusion, which drugs were used, which setup of the infusion volume and speed schedule, checking and certifying that all these data are in accordance with the prescription, and sending any adverse event to a Monitoring Unit (MU).

In a first modality, a DIE is attached externally to a device, without any data connection. In this way, the DIE attached to the equipment allows to certify and record interactions with other living things ad or lifeless things, allowing to prove and certify who handled the equipment and the supplies used, for this all living things ad or lifeless things have electronic devices attached.

In a second modality, the electronic device (DIE) is internally attached to a device without connectivity through the interception of the communication cable connected to the data bus of the interface used to externalize the information to the user, allowing the DIE to interpret the data sent to the equipment interface, implementing to it the ability to certify and record interactions with living things and or lifeless things, and also send, through the electronic device (DIE), all the information presented on the interface to the Central Processing Unit (CPU), which processes and links data to living things and or lifeless things to send this information to electronic devices (DIUs) and (DIEs), applications and the Monitoring Unit (MU).

In a third modality, an electronic device (DIE) is attached to equipment without connectivity through a specific connection cable for communication with the electronic device (DIE), allowing that said electronic device (DIE) to interpret all data sent by the cable connection, implementing to it the ability to certify and record interactions with living things and or lifeless things, and also send, through the DIE, all information received from the equipment to the Central Processing Unit (CPU), which processes and links the data with living things and or lifeless things and sends this information to electronic devices (DIUs) and (DIEs), applications and Monitoring Unit (MU).

In a fourth modality, an electronic device (DIE) is coupled to equipment with native connectivity and said equipment communicates with the CPU through an API, implementing the ability to certify and record interactions with live things and lifeless things, and also send, through the CPU, information about its operation. The CPU links the events sent by the equipment and the interactions of other electronic devices (DIUs) and / or (DIEs) established with the electronic device (DIE) coupled to the equipment.

Equipments with DIE coupled acquire the ability to remotely send to the manufacturer, information on operating conditions, such as usage time, battery level, number of operations, supplies used in each operation, setup used in each operation, calibration data , whether they are on or off, whether they are operating connected to the main power or operating on battery, quantity and type of adverse events generated, as well as data on the interactions established with other DIUs and DIEs, particularly useful for monitoring performance and controlling leased or lending equipment, allowing you to control inventories and other data for each contract.

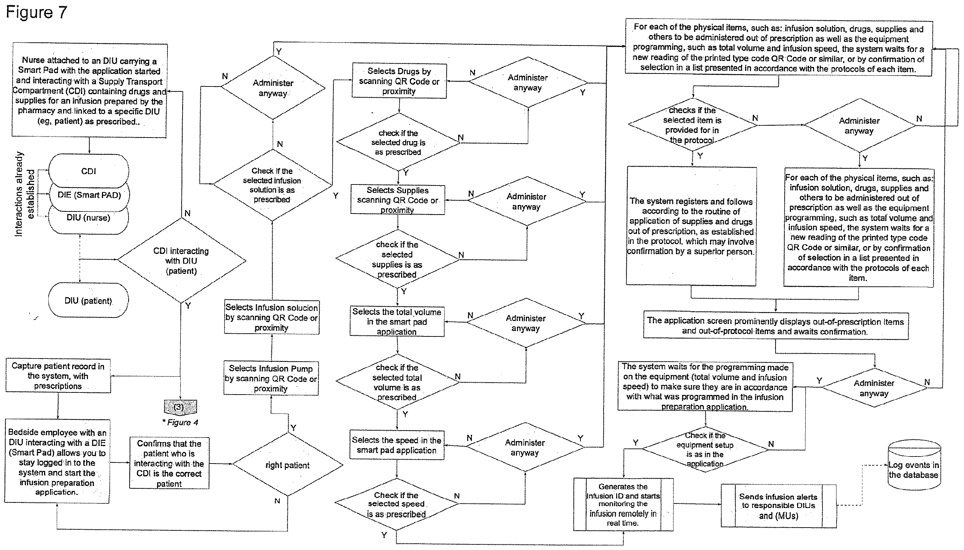

As shown in figure 7, it exemplifies the routine of using equipment such as in this case, an infusion or nutrition pump (with an electronic device (DIE) attached) used to administer a drug (with an electronic device (DIE) attached), according to a prescription for a patient (with an electronic device (DIU) attached). The infusion or nutrition pump used in the process is linked to establish interaction with the drug's DIE and / or with the prescription patient's DIU and / or with the professional DIU responsible for the administration and programming of the equipment. These interactions and links with the process are recorded at the Central Processing Unit (CPU), which sends all information related to the infusion process to the monitoring units (MU) and / or to the electronic devices (DIUs) responsible for the event, such as: infusion speed, volume already infused, medication used, malfunction alarms, end of infusion alarms and all other data generated by the equipment.

Optionally, in the interface of the electronic device coupled to a lifeless thing (DIE), are presented the DIUs enabled to operate said lifeless thing, as well the presentation of information about the functioning of that lifeless thing.

In the interface disposed in the electronic devices (DIU) and (DIE) a user can access system functionalities, such as sending an alert to other electronic devices of users (DIUs) previously registered in the central processing unit (CPU) database and / or send a message to mobile phones registered in the central processing unit (CPU) database. This functionality is particularly useful, for example, in situations where a user with an electronic device (DIU) attached needs emergency assistance from a third party.

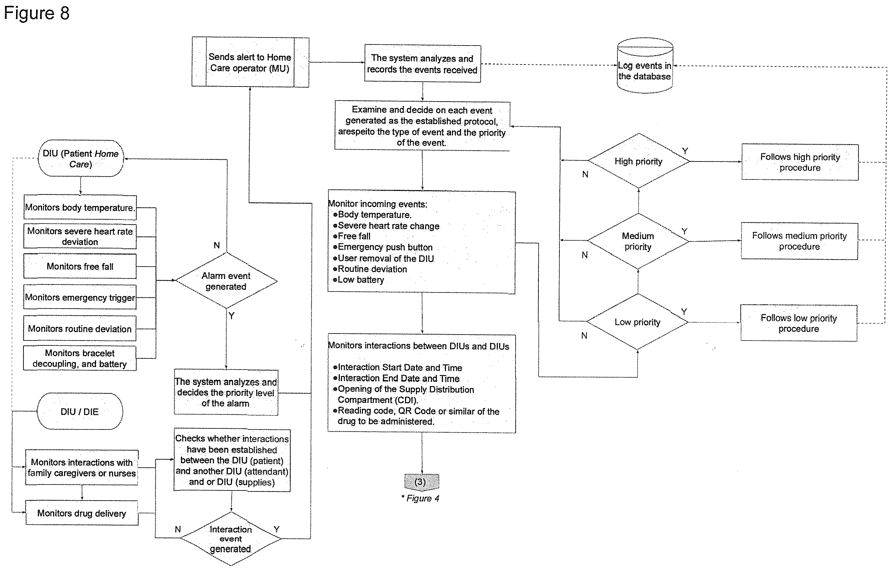

Figure 8 presents a system of care control and monitoring of patients in Home Care, where it is possible to identify the interactions established between electronic devices (DIEs) attached to lifeless things / or electronic devices (DIUs) attached to living things like users and monitor the physical and physiological parameters of the electronic devices (DIUs) attached to users, making it possible to manage the interactions established with a patient and for how long it lasted, record the delivery of supplies, keep monitoring their physiological parameters, and monitor their routines through the accelerometer data, allowing to anticipate actions, in case of alerts sent by the electronic device (DIU) attached to the patient in Home Care. As an example, we can cite data received by the Central Processing Unit (CPU) that indicate a fall of the patient without data being received indicating that he was standing. In this case, the system automatically triggers the emergency procedures defined in its protocol. As another example we can mention a caregiver with an electronic device (DIU) attached, which establishes interaction with a Fixed DIE attached to the entrance area of a patient's home, recording the moment that this caregiver arrived at the patient's home, then establishes interaction with the patient for 15 minutes, and 60 minutes later the interaction with the fixed DIE is ended by leaving the caregiver, recording his departure from the patient's residence, indicating that of the 60 minutes present in the residence, only 15 were actually dedicated to the patient. Another facility generated by the sensing of body data and the identification of interactions is to allow the identification of a patient's reactions due to the taking of a medication that may cause some adverse reaction, such as a drastic change in heart rate or temperature. The system immediately generates an alert to the responsible DIUs.

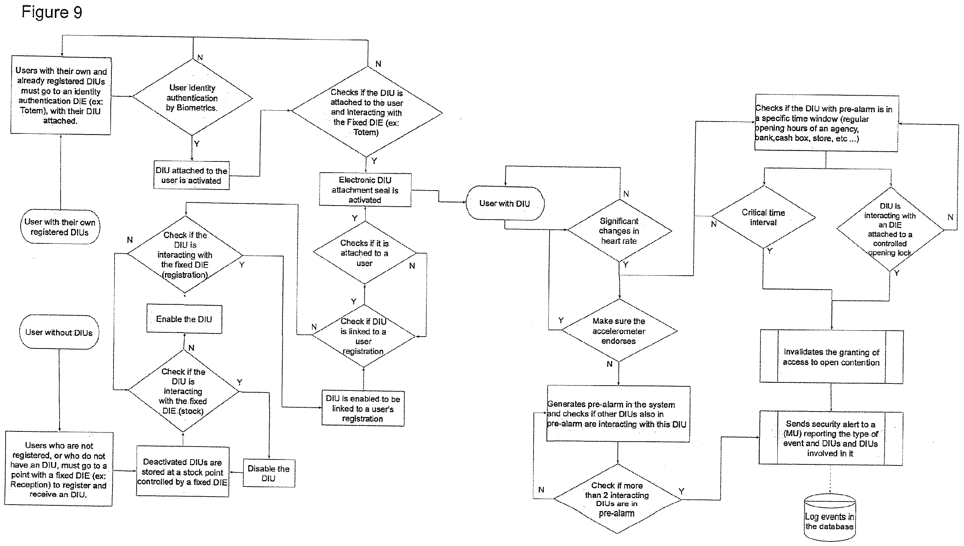

As shown in figure 9, physiological changes of a user or a group of users can be monitored, generating alerts due to physiological changes certified by an accelerometer that defines them normal or not depending on the physical activity from data obtained in function the modeling of types of actions (exercise, rest, rapid deceleration, rapid acceleration, overturning, among others). In this way, the interactions established between DIUs and / or DIEs combined with the physiological data of a user or a group, associated with the data from the accelerometer, can indicate a series of critical safety events sending to the Central Processing Unit (CPU) that processes and sends alert messages to Monitoring Units (MU) or to previously defined contacts (public or private emergency emergency services, for example). An electronic device (DIU) attached to a user sends data to the Central Processing Unit (CPU) that records a significant change in heart rate, without the corresponding activity on the accelerometer that would certify this as normal or not, which may indicate an emergency situation. A collective change in heart rate without corresponding accelerometer activity in interacting DIUs may indicate an emergency. A professional driver with an (DIU) that has a GPS or API connectivity with any other global positioning device, shows an emergency situation due to the relationship between the physiological functions and the accelerometer data, whose modeling can indicate, accident, rollover, assault, sudden collision, sleep, among others, allowing immediate action of emergency measures.

Another facility generated by the sensing of corporeal data and the identification of interactions is to allow the identification of critical events based on collective physiological changes. For example, changing the collective heart rate of a group of employees who are using DIUs with established interaction can indicate an adverse safety event, allowing them to alert security teams to a more immediate reaction.

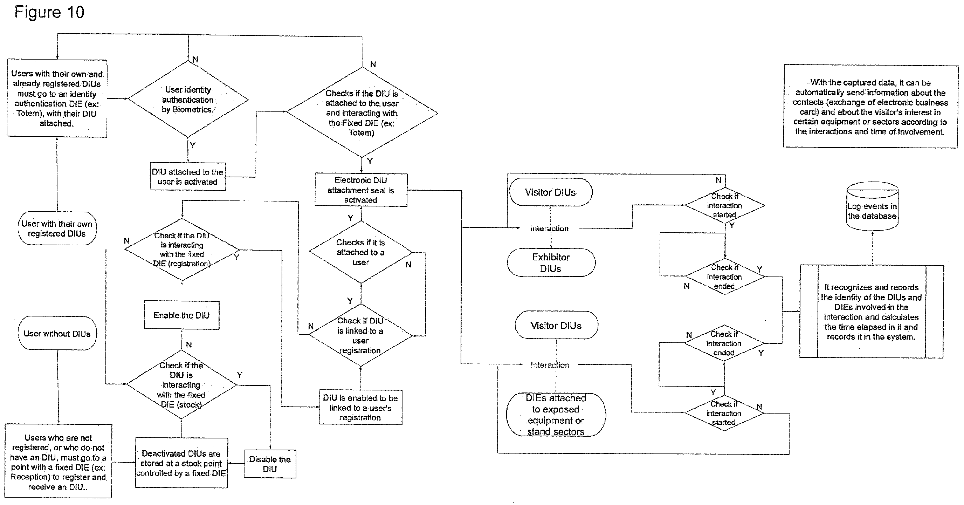

As shown in figure 10, the electronic devices (DIUs) and (DIEs) allow, for example, tracking the behavior of visitors in an event, their relationships with exhibitors, and their interests in specific areas of a stand or in exposed equipment. Considering that the visitors and exhibitors are with DIUs and the equipment and areas to be tracked have DIEs or fixed DIEs, it is possible to evaluate the behavior of the visitors according to the established interaction and its duration.

Electronic devices attachable to lifeless thing (DIE) can be installed indoors (being referred to in the present invention patent as fixed DIEs), such as in furniture or in fixed structures (for example, chambers refrigerated), in order to record the interactions with other electronic devices (DIUs) and / or (DIEs) that are in its coverage area and send the data to the Central Processing Unit (CPU), which checks, valid, alerts and records interactions.

For example, a fixed DIE installed in an exam and/or procedure room or clinic identifies an DIU nearby and authenticates it, so that the central processing unit (CPU) certifies the presence of a user with an DIU, allowing attesting and proving the physical presence of this user with date and time and the remained time in interaction with the fixed DIE.

Optionally, electronic devices for lifeless things (DIEs) are installed in physical or virtual containment means or equipment to control access to restricted areas, such as turnstiles, doors, gates, software access screens, said electronic devices (DIE) that sends proximity data to the Central Processing Unit (CPU) that identifies an interaction with an electronic device (DIU) attached to a user and linked in the system, allowing or not allowing access to the restricted area, according to authorizations defined in the Central Processing Unit (CPU).

Additionally, an electronic device (fixed DIE) can control the presence of other electronic devices (DIEs) through the establishment of interaction between them, and record the exit or entry of a DIE from a certain area, as well as the Central Unit. processing (CPU) can, depending on the beginning and end of the interactions registered between a Fixed DIE and other DIEs, generate alerts and block the activation of containment equipment from instructions defined in the Central Processing Unit (CPU), ensuring, thus, blocking the passage through the containment equipment, for example, from an equipment with an electronic device (DIE) attached.

The system and method described in the present invention patent allows to implement the automatic analysis of the protocols registered in the system in function of the patient's evolution reactions, comparing the results obtained, for each protocol standard and relating the results by level of effectiveness, being able to suggest the best protocol to be used or automatically change the protocol according to the best results. As an example, we can imagine that in a hospital A it is adopted as a protocol that the administration of a certain medication more than two hours late the dose should be omitted, while in hospital B, for the same medication, a different protocol is applied, allowing administration of the dose even 2 hours late and not rescheduling the next dose. Given the possibility of being able to follow the patient's evolution, through his physiological patterns, and allowing the patient's own pain perception and evolution data to be entered through the electronic device interface (DIU) and / or by the application of use the bedside professional, the system compares the two protocols, using the records database and defines the best performance protocol.

## Claims

1. SYSTEM OF MONITORING AND MANAGEMENT OF INTERACTIONS BETWEEN LIVING THINGS AND / OR LIFELESS THINGS
**characterized by** comprising:
**a)** two or more electronic devices (DIU) and / or (DIE) equipped with an identification code (ID) linked, respectively, to the profile of a user or to the characterization of a lifeless thing, an electronic circuit board, a processor, a wireless communicator, a decoupling sensor, firmware and a power source, said electronic devices (DIU) and / or (DIE) that interact with each other through communication protocols or just read emitted signals, without necessarily establishing communication, but just because they are in physical proximity;
**b)** a Central Processing Unit (CPU) that receives the messages with information about the signals read by the electronic devices (DIU) or (DIE) and processes in an Event Generating Process (EGP) that compares and submits the registered logic and defines the interactions between living things and or lifeless things, recording in the database, which is accessed by the Server Process (SP) which analyzes the interaction information and acts in accordance with the system directives, returning information, commands, actions and instructions to the electronic devices (DIUs) and / or (DIEs), applications and Monitoring Unit (MU);A Monitoring Unit (MU) that receives data from the central processing unit (CPU) and / or from a Server Process (SP) to allow the monitoring of interactions and other alerts generated in the system.

2. SYSTEM, according to claim 1, **characterized by** the fact that the Central Processing Unit (CPU) defines, based on a set of instructions, which electronic devices (DIUs) and / or (DIEs) should be considered that there is a beginning of interaction and an end of interaction and how much time elapsed between the beginning of an interaction and the end of this interaction.

3. SYSTEM, according to claim 1, **characterized by** comprising an electronic device (DIE), coupled to a conventional electronic device, which communicates or reads the signals from electronic devices (DIUs) and / or (DIEs) that are within reach and communicates with the Central Processing Unit (CPU) through the operating system itself or through API.

4. SYSTEM, according to claim 1, **characterized by** the fact that the link with a living and / or a lifeless thing, to an electronic device (DIE) or (DIU) is completed in a specific location provided with a fixed electronic device (Fixed DIE) and configured in the system as a registration point and device link, said link made by a user with an attached DIU and with the authority to register at the Central Processing Unit (CPU).

5. SYSTEM, according to claim 1, **characterized by** the fact that the linking of a living or lifeless thing to an electronic device (DIU) or (DIE) is made in an automated attendance totem, equipped with a Fixed DIE registered in the system as an automatic registration point and device linking, by a user registered in the system and presenting an attached DIU and without proven authenticity, through a method of personal recognition that maintains the integrity of the linking.

6. SYSTEM, according to claims 4 or 5, **characterized in that** the linked electronic device (DIU) or (DIE) is activated when this device (DIU) or (DIE) is coupled to a living or lifeless thing and establishes an interaction with the linking point and device registration.

7. SYSTEM, according to claim 1, **characterized by** the fact that the decoupling sensor of the electronic devices (DIU) and (DIE) identifies the removal or uncoupling of an electronic device (DIU) or (DIE) from your living thing or lifeless thing respectively, invalidating the ability to authentication of this electronic device (DIU) or (DIE) and generate alerts to electronic devices registered in the system to receive alerts of decoupling and invalidation.

8. SYSTEM, according to claim 1, **characterized by** the fact that the electronic devices (DIU) and (DIE) have a physical button or a means of selection on the interface of the electronic device (DIU) or (DIE) that sends a request for assistance to other electronic devices (DIUs) and / or (DIEs) and / or a Monitoring Unit (MU), according to the routine established in the Central Processing Unit (CPU).

9. SYSTEM, according to claim 1, **characterized by** the fact that the electronic device attached to the user (DIU) presents sensors of physiological parameters monitored by the Central Processing Unit (CPU) that generates alerts to other devices (DIUs) when identifying changes in the parameters, according to a pre-established standard.

10. SYSTEM, according to claim 1, **characterized by** the fact that the electronic device (DIU) attached to a user, presents accelerometer-type motion sensors that send data to the Central Processing Unit (CPU) that processes and analyzes the data received, comparing with other user parameters and with the standard behavior routine.

11. SYSTEM, according to claim 1, **characterized by** the fact that the electronic devices (DIU) and (DIE) present an interface that serves as a way of interaction with the user of an electronic device (DIU) or (DIE) to send and receive activations, information, instructions, audible and / or visual alerts.

12. SYSTEM, according to claim 1, **characterized by** the fact that the supply destined to a user attached to an electronic device (DIU) is inserted in a Supplies Distribution Compartment (CDI) attached to an electronic device (DIE) with an electronic seal, this supply is provided with an identified code that can be read through a code reader.

13. SYSTEM, according to claim 1, **characterized by** the fact that the supply designated for a user with an electronic device (DIU) attached, has an electronic device (DIE) attached to this supply.

14. SYSTEM, according to claim 1, **characterized by** the fact of presenting an electronic device (DIE) attached to an equipment without connectivity.

15. SYSTEM, according to claim 1, **characterized by** the fact that an equipment without connectivity has an electronic device (DIE) connected to the communication cable of the data bus used to externalize the information to the user interface of this equipment or to connect the electronic device (DIE) on a specific cable for communication with the equipment.

16. SYSTEM, according to claim 1, **characterized by** the fact that it comprises an electronic device (DIE) attached to an equipment with native connectivity.

17. SYSTEM, according to claim 1, **characterized by** the fact that it comprises a fixed DIE attached to a civil structure or furniture that identifies a nearby DIU and authenticates it, so that the central processing unit (CPU) certifies the presence of a user with an electronic device (DIU) attached and the time that remained in interaction with the fixed DIE.

18. METHOD OF MONITORING AND INTERACTION MANAGEMENT BETWEEN LIVING THINGS AND / OR LIVELESS THINGS using the system claimed in 1, **characterized by** understanding the interaction, through physical proximity, of two or more electronic devices (DIU) and / or (DIE) whose signals are sent to the Central Processing Unit (CPU) which, based on a set of instructions, verifies the beginning of the interaction and the end of the interaction and the time elapsed between the beginning of an interaction and the end of this interaction.

19. METHOD, using the system claimed in 12, **characterized by** the fact that the administration of a supply destined for a user with an electronic device (DIU) attached comprises:
**a)** linking the scannable identifier of a supply to a user (with a DIU) must be done in interaction with a Fixed DIE configured in the system as a supply distribution area and by a user (with a DIU) enabled in the system for this event;
**b)** linkage of the CDI (with a DIE) that carries the supply to the destination user (with a DIU);
**c)** registration of all links of electronic devices DIUs and DIEs in the database of the Central Processing Unit (CPU);
**d)** established the end of interaction between the electronic device (DIE) attached to the Supply Distribution Compartment (CDI) and the Fixed DIE installed in the supply distribution sector, and certified that the state of the CDI electronic seal is closed, the Unit Processing Center (CPU) generates an alert to electronic devices (DIUs) linked to the user receiving the supply (holder of a DIU) to indicate the availability of the supply;
**e)** CDI with the attached electronic device (DIE) is transported by a user with an electronic device (DIU) and enabled for this transport, such interaction being registered at the Central Processing Unit (CPU);
**f)** the approximation of the CDI containing the electronic device (DIE) with the electronic device (DIU) of the patient linked to that CDI generates a correct patient certification;
**g)** Event Generating Process (EGP) checks the interaction between the electronic device of patient (DIU) and the electronic device (DIE) of the CDI and records in a database and checks whether these electronic devices (DIU) and (DIE) are associated and if the time of the interaction is in accordance with that registered in the specific database;
**h)** if the data is certified, the Server Process (SP) authorizes the opening of the electronic seal of the Supply Distribution Compartment (CDI);
**i)** scanning of the identifier code, attached to the supply, using an application installed on a mobile device that must have an electronic device (DIE) attached if it is unable to interact with the electronic devices (DIUs) natively.

20. METHOD, using the system claimed in 13, **characterized by** the fact that the administration of a supply destined for a user with an electronic device (DIU) attached comprises:
**a)** the linking of a supply (with a DIE), to a user (with a DIU), must be done in interaction with a Fixed DIE configured in the system as a supply distribution area and by a user (with a DIU) enabled in the system for this event;
**b)** registration of all links of electronic devices DIUs and DIEs in the database of the Central Processing Unit (CPU);
**c)** Central Processing Unit (CPU) generates an alert to electronic devices (DIUs) linked to the user responsible the supply (holder of a DIU) to indicate the availability of the supply;
**d)** supply with the attached electronic device (DIE) is transported by a user with an attached electronic device (DIU) enabled for this transport, such interaction being recorded at the Central Processing Unit (CPU);
**e)** the approach of supply with an electronic device (DIE) attached to a patient with an electronic device (DIU) attached and linked to that DIE generates a correct patient certification;
**f)** Event Generator Process (EGP) checks the interaction between the electronic device (DIU) attached to a patient and the electronic device (DIE) attached to the supply, records it in the database and checks whether there is an association between such electronic devices (DIU) and (DIE) ) and if the interaction time is according to the specific forecast registered in the database.

21. METHOD, according to claims 19 and 20, **characterized by** the fact that the electronic device (DIU) attached to a user authorized to administer the input to the recipient user (with an attached DIU) receives guidance about the input's administration, through the interface provided on your electronic device (DIU).

22. METHOD, according to claim 19, **characterized by** the fact that the Central Processing Unit (CPU) generates an alert if the reading of the supply code to be administered is non-compliant.

23. METHOD, according to claims 19 and 20, **characterized by** the fact that the Central Processing Unit (CPU) generates an alert if the electronic device (DIU) attached to a user to whom the supply is being delivered is not associated with the DIE of the Supply Distribution Compartment (CDI) or the supply DIE.

24. METHOD, according to claims 19 and 20, **characterized by** the fact that the CPU generates an alert if the DIU of the transport user of the Supply Distribution Compartment (CDI) or of the supply is not enabled for the event.

25. METHOD, according to claims 19 and 20, **characterized by** the fact that the Central Processing Unit (CPU) generates an alert if there is a physical distance between a Supply Distribution Compartment (CDI) or a supply attached to an electronic device (DIE) and the electronic device (DIU) authorized for the transport of that Supply Distribution Compartment (CDI) or supply, unless they are also interacting with one or more destinations (fixed DIE or patient DIU) linked to said electronic device (DIE).

26. METHOD, according to claims 19 and 20, **characterized by** the fact that the Central Processing Unit (CPU) automatically registers the routine of administering a supply to a recipient user with an electronic device (DIU) from the first administration.

27. METHOD, according to claims 19 and 20, **characterized by** the fact that the rescheduling of the administration of a supply occurs automatically whenever there is a breach of the schedule window defined as normal in relation to the prescription to a recipient of the supply.

28. METHOD, according to claims 19 and 20, **characterized by** the fact that the CPU generates a non-prescription alert in an event of attempt to administer a non-prescribed input to a specific user with a DIU, triggering a defined protocol, sending an alert through the interface of the electronic device (DIU) and (DIE) and / or application, if the interaction of the electronic devices (DIU) and / or (DIE) of those involved is maintained.

29. METHOD, according to claim 18, **characterized by** the fact that the CPU generates an alert on the interface of electronic devices (DIU) and / or (DIE) and or in the application when the interaction between an electronic device (DIU) attached to a user is identified and an electronic device (DIE) attached to a lifeless thing with some restriction to the user.

30. METHOD, according to claims 19 and 20, **characterized by** the fact that the supplies configured in the database of the Central Processing Unit (CPU) as having a specific routine require Double Check, sending an alert through the electronic device interface (DIU) and (DIE) and / or application, as well as presenting on the interfaces of these the routine to be fulfilled, which must be confirmed by the user receiving the alert.

31. METHOD, according to claims 19 and 20, **characterized by** the fact that the return to the stock of any supply that has not been used for any reason is controlled, registering the return at the Central Processing Unit (CPU) and being able to generate an alert informing the reason.

32. METHOD using the system described in claim 14, **characterized by** the fact that the electronic device (DIE) attached to the equipment without connectivity certifies and records interactions with other holders of electronic devices (DIUs) and / or (DIEs) nearby.

33. METHOD using the system described in claim 15, **characterized by** the fact that the electronic device (DIE) processes and links data to living and lifeless things and sends this information to electronic devices (DIUs) and (DIEs), applications and the Monitoring Unit (MU).

34. METHOD using the system described in 16, **characterized by** the fact that the equipment communicates with the Central Processing Unit (CPU) through an API, sending information about its operation and allowing to certify and record the interactions with living things and lifeless things.

35. METHOD, according to claims 32 to 34, **characterized by** comprising the steps:
**a)** association of the equipment DIE with the patient's DIU and the supply DIE to be used in the equipment and / or with the DIU attached to the professional responsible for the administration and programming of the equipment;
**b)** registration of links in the Central Processing Unit (CPU);
**c)** CPU sends the associations to the monitoring units (MU) and / or to the electronic devices (DIUs) responsible for the event.

36. METHOD, according to claim 34, **characterized by** the fact that the interface of the electronic device coupled to a lifeless thing (DIE) presents the electronic devices (DIUs) enabled to operate this lifeless thing, as well as the functioning information of the same.

37. METHOD, according to claim 18, **characterized by** the fact that the interface disposed in the electronic devices (DIU) and (DIE) allows the triggering of emergency alerts to electronic devices of users (DIUs) previously registered in the database of the central unit of processing (CPU) and / or allow sending a message to mobile phones registered in the central processing unit (CPU) database.

38. METHOD, according to claim 18, **characterized by** the Central Processing Unit (CPU) to identify the interactions established between electronic devices attached to lifeless things (DIEs) and / or electronic devices attached to users (DIUs) and monitor the physical and physiological parameters through electronic device (DIU) attached to users.

39. METHOD, according to claim 18, **characterized by** the fact that the physiological changes of a user or a group of users are defined as an emergency when analyzed together with the data from an accelerometer, whose modeling of the types of movement, and physiological changes certified by CPUs can generate alerts.

40. METHOD, according to claim 18, **characterized by** the fact that a DIE is attached to containment means or equipment, this electronic device (DIE) that sends the proximity data to the Central Processing Unit (CPU) that identifies an interaction with a electronic device attached to a user (DIU) in order to allow or not allow access to the restricted area, according to authorizations defined in the Central Processing Unit (CPU).

41. METHOD, according to claim 18, **characterized by** the fact that it allows the automatic analysis of the protocols registered in the Central Processing Unit (CPU) related to the patient's evolution reactions, comparing the results obtained, for each protocol standard, and relating the results by level of effectiveness.
